# EUROPEAN PATENT SPECIFICATION

(11) **EP 0 110 280 B1**
(45) Date of publication and mention of the grant of the patent: **12.10.1994**
(21) Application number: 83111615.7
(22) Date of filing: 21.11.1983
(51) Int. Cl.: C07D 499/04, C07D 205/08, C07F 7/08, A61K 31/43

(54) **Process for the production of penem compounds**
Verfahren zur Herstellung von Penem-Verbindungen
Procédé de préparation de dérivés de pénème

(30) Priority: 29.11.1982 US 445295; 17.01.1983 US 458511; 28.01.1983 US 461845; 31.01.1983 US 462723
(43) Date of publication of application: 13.06.1984
(60) Divisional application: 89113483.5
(73) Proprietor: SCHERING CORPORATION, Kenilworth New Jersey 07033 (US)
(72) Inventor: Girijavallabhan, Viyyoor Moopil, Parsippany New Jersey 07054 (US); Ganguly, Ashit Kumar, Upper Montclair New Jersey 07043 (US); Pinto, Patrick Anthony, Mine Hill New Jersey 07801 (US); Versace, Richard William, Ringwood New Jersey 07456 (US); McCombie, Stuart Walter, West Orange New Jersey 07052 (US)
(74) Representative: Ritter, Stephen David

(56) References cited:
- EP-A- 002 210
- EP-A- 013 662
- EP-A- 024 832
- EP-A- 046 363
- EP-A- 058 317
- EP-A- 069 373
- EP-A- 070 204
- EP-A- 087 792
- EP-A- 0 078 026
- EP-A- 0 080 162
- DE-A- 3 224 055
- FR-A- 2 477 547
- FR-A- 2 483 925
- GB-A- 207 453
- GB-A- 2 042 514
- GB-A- 2 042 520
- GB-A- 2 105 329
- CHEMICAL ABSTRACTS, vol. 97, no. 5, 2nd August 1982, page 552, no. 38722c, Columbus, Ohio, US

## Description

This invention relates to processes for preparing 6-hydroxyalkyl-2-substituted thio-penem-3-carboxylic acids, and their salts and esters (referred to herein as penems) to certain novel penems and pharmaceutical compositions containing them.

Penems, a recent addition to the family of synthetic β-lactams, possess potent anti-bacterial activity. They have, hitherto been prepared by laborious, time consuming, multistep processes which result in low yields and are thus uneconomical.

Typical prior art procedures are described in GB-A-2 042 514, GB-A-2 074 563, GB-A-2 042 520 and EP-A-0 058 317.

According to one aspect of the present invention we provide a process for the preparation of penem compounds of formula I 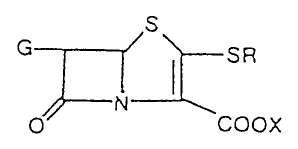
having the stereo configuration 5R,6S, wherein R is hydrogen; lower alkyl; loweralkyl substituted with one or more substituents independently selected from halogen, hydroxy, lower alkoxy, cyano, oxo, carb(lower)alkoxy, carbamoyl, N-lower-alkyl-substituted-carbamoyl, N,N-diloweralkyl substituted-carbamoyl, amino, loweralkylamino, diloweralkylamino, lower alkanoylamino, aminoacylamino, alkylthio, arylthio, heterocyclic, heterocyclicthio, 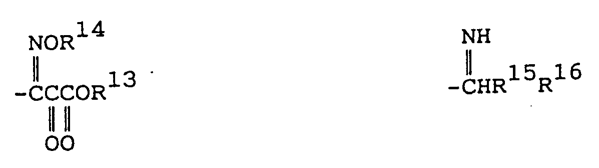
(wherein R¹⁴ and R¹³ are independently hydrogen or lower alkyl and R¹⁵ and R¹⁶ are each lower alkyl or R¹⁵ and R¹⁶ together with the nitrogen to which they are attached form a heterocyclic ring of 3 to 6 members) butanolidyl, aryl, heteroaryl or aryl or heteroaryl substituted with 1 to 4 substituents independently selected from loweralkyl, hydroxy, lower-alkoxy, halogen, haloloweralkyl, loweralkylthio, amino, lower-alkylamino, diloweralkylamino, carboxyloweralkyl, nitro, and cyano;
aryl or aryl substituted with 1 to 4 substituents independently selected from loweralkyl, hydroxy, lower alkoxy, halogen, haloloweralkyl, loweralkylthio, amino, loweralkylamino, diloweralkylamino, carboxyloweralkyl, nitro, cyano, aminoalkyl and carboxy; and
heteroaryl or heteroaryl substituted with 1 to 4 substituents independently selected from loweralkyl, hydroxy, lower alkoxy, halogen, haloloweralkyl, lower alkylthio, loweralkylsulfonyl, amino, loweralkylamino, diloweralkylamino, carboxyloweralkyl, nitro and cyano,
wherein the term "halogen" means fluoro, chloro and bromo,
the term "loweralkanoylamino" means the group 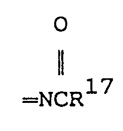
wherein R¹⁷ is lower alkyl,
the term "aryl" means aromatic groups of 6 to 10 carbons,
the term "heterocyclic" means cyclic groups of 5 or 6 ring atoms wherein 1 to 4 ring atoms are selected from nitrogen, sulfur and oxygen,
the term "heteroaryl" means aromatic heterocyclic groups of 5 to 10 ring atoms wherein 1 to 3 ring atoms are selected from the group consisting of nitrogen, sulfur and oxygen,
and the term loweralkyl means branched and straight-chain alkyl groups of 1 to 6 carbon atoms.

G is hydroxy C₁ to C₆ alkyl, X is hydrogen, a pharmaceutically acceptable salt forming group, a pharmaceutically acceptable ester group or a carboxy protecting group, and the tautomers thereof when R is hydrogen, characterised in that a compound of formula VI 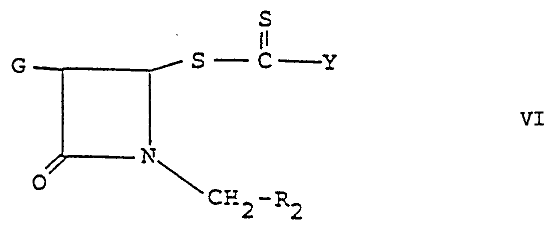
wherein R₂ is a protected carboxy group and Y is a leaving group, is subjected to intramolecular cyclization in an anhydrous inert organic solvent in the presence of non-nucleophilic strong base whereby a tautomeric compound of formulas Va and Vb is obtained 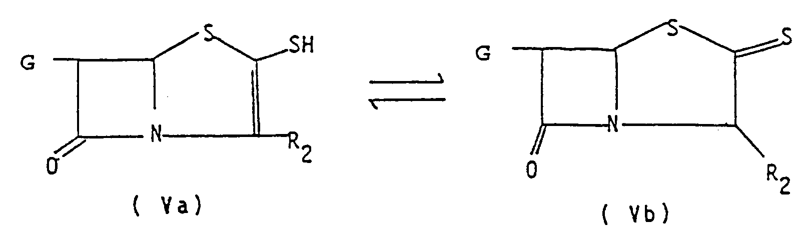
in which R₂ is as defined above, and that, if desired, such tautomeric compound is transformed into a compound of formula I wherein R represents an organic radical by introducing such R by known conventional means.

Compounds of VI, Va and Vb defined above are novel and provide further aspects of the invention.

Among the methods used for transforming a tautomeric compound of formula [( Va), ( Vb)] into another compound of formula I it is preferred to use one or other of the following:
(i) reaction with a compound of the formula

   RZ

   in which R is an organic group, as defined in processes (a), (b) and (c) and Z is a leaving group. Typical leaving groups Z are halides, e.g. chlorine, bromine and iodine, hydroxyl and trichloromethylsulfonyl;
(ii) olefine addition, for example using a 1,2-unsaturated unsubstituted or substituted C₂-C₆ alkylene whereby a compound of formula I in which R is an unsubstituted or substituted C₂-C₆ alkyl group is produced;
   This type of reaction is useful for example in the production of 2-alkylthio substituted penems. By using olefines of the formula

   R₃CH=CH₂

   in which R₃ is hydrogen or lower alkyl e.g. methyl, ethyl or propyl, compounds of formula I in which R is ethyl, propyl, butyl and pentyl can be obtained;
(iii) reaction with a substituted or unsubstituted 1,2-epoxy C₂-C₆ alkane, whereby a compound of formula I in which R is a C₂-C₆ alkyl group having a hydroxy group attached to the second carbon atom from the sulphur atom and optionally carrying one or more other substituents is obtained.
   This type of reaction can for example, thus be used for producing compounds of formula I in which R is e.g. 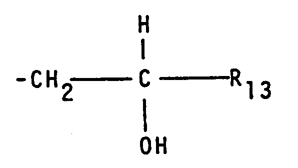 in which R₁₃ is hydroxyloweralkyl or carboxyloweralkyl in which the carboxy group may be esterified or salified. By using glycidol a compound of formula I in which R is 2,3-dihydroxy-propyl can be obtained for example; glycidic acid and alkali metal glycidates lead to compounds of formula I in which R is 2-hydroxy-3-carboxyl propyl and alkali metal salts thereof respectively -

For convenience the tautomeric compound [(Va), (Vb)] will be referred to below simply as the compound V.

The process defined above is novel and inventive and is capable of producing known and novel penems in high yield.

Suitable carboxyl protecting groups are those which can be removed under conventional conditions without reacting with other functional groups present on the β-lactam, for example allylic, cyanoloweralkyl, loweralkylsilylloweralkyl, sulfone esters, p-nitrobenzyl and trichloroethyl. For instance the preferred protecting group in R₂ is allyl; and in R₁ and R₂, in compound II, the preferred protecting groups are preferably respectively loweralkylsilylloweralkyl and allyl.

Unless otherwise stated, the term "loweralkyl" includes branched- and straight-chain alkyl groups of from 1 to 6, preferably 1 to 4, carbon atoms and includes, for example, methyl, ethyl, n-propyl, isopropyl, t-butyl, pentyl and hexyl.

The term "removable hydroxy protecting group" means any such group conventionally used for this purpose, with the only requirement being compatability with the hydroxy substituent on the penems and β-lactam intermediates and removability utilizing elemental zinc or any other conventional agent for this purpose which will not adversely affect the penem or β-lactam intermediates structure. The preferred hydroxy protecting groups include trichloroethoxycarbonyl, dimethyltributylsilyl, trimethylsilyloxycarbonyl and trimethylsilyl.

The process of this invention can be used to produce penems of any desired stereochemistry. The most preferred stereochemistry of the final penem compounds is (5R,6S,8R), less preferred is (5R,6R,8S). Final penems of desired stereochemistry can be obtained by selection of starting material of appropriate stereochemistry.

Cyclization of the compound of formula VI into the tautomeric compound V is typically carried out in an anhydrous inert organic solvent, for example, tetrahydrofuran, and a non-neuclophilic strong base, for example, lithium diisopropyl amide (LDA) and lithium di-(trimethylsilyl)amine added to the system to effect cyclization. Usually cyclization will be carried out at from about -50°C to -100°C and preferably at about-70°C and will generally be complete within about 5 minutes to 24 hours. Usually an essentially equimolar amount of the strong base will be used.

The transformation of a tautomeric compound V into another compound of formula I, is typically carried out in an anhydrous inert organic solvent for example, tetrahydrofuran, ethylether or dioxane at temperatures ranging from about -10°C to about 45°C e.g. 10°C to 45°C with room temperature (about 25°C) being preferred.

In the case of this transformation being carried out on a tautomeric compound V it will be noted that deprotection of the 8-position hydroxy group may previously be conducted, using suitable reagents, as discussed below, in such solvents and in a similar temperature range. Thus the transformation may be conducted as a continuation of such preceding deprotection step namely without isolation of the tautomeric compound V. Thus the same solvents will be used in both steps and it will also be usual to carry out reactions at about the same temperature, or at least in the same above mentioned temperature range in both steps. If the tautomer is, first, isolated, different solvent and temperature can be used in this step compared with the preceding step but, nevertheless, these will preferably be the same.

Transformation using a compound RZ is generally carried out in the presence of a base or acid acceptor. Such bases and acid acceptors known in the art for this reaction can be used e.g. inorganic bases such as alkali metal carbonates or bicarbonates, or organic bases such as triethylamine. Where tetrabutylammonium fluoride has previously been used (to remove R₁ in process (a) ) and remains in the reaction solution this also will function as a base in this reaction.

The alkylation step can, for example, be carried out, in one embodiment, by the reaction of a compound X of formula 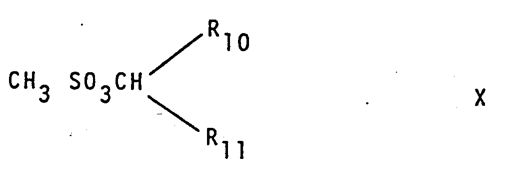
In which R₁₀ and R₁₁ are chosen so that the group -CHR₁₀R₁₁ corresponds to R in Formula I, with the tautomeric compound of formula V. This particular reaction will typically be carried out in a suitable organic solvent, for example, tetrahydrofuran. An essentially equimolar amount of an acid acceptor, for example an inorganic carbonate, facilitates the reaction. Typical reaction temperatures are in the range from about -5°C to about 30°C, and the reaction is generally complete within 1 to 24 hours.

Olefin addition is generally carried out using a radical initiator for example ABN[2,2'-azobis(2-methylpropionitrile)].

The removal of the protecting group from the protected carboxyl group R₂ can be carried out by conventional procedures selected according to the identity of the protecting group. The preferred protecting group is allylic, preferably allyl, and its removal can, in general be effected under catalytic conditions in the presence of a base, preferably by utilizing procedures described in our European Patent Application Publication No. 0013663. Thus the allylic groups are preferably removed by utilizing a suitable aprotic solvent, such as tetrahydrofuran, diethyl ether or methylene chloride, with an alkali metal alkylcarboxylate, preferably potassium or sodium 2-ethylhexanoate (to give the alkali metal penem salt, preferably the sodium or potassium penem salt, directly) or carboxylic acid, preferably 2-ethylhexanoic acid (to give the penem free-acid) and a mixture of a palladium compound and triphenyl phosphine as a catalyst. Most preferably this step procedes with the removal of the allylic protecting group and the formation of the alkali metal salt of the penem in situ.

If the product is a zwitterion deprotection of the allylic group requires only the catalyst and any mild neucleophile (e.g. H₂O or alcohol).

This procedure will usually yield the free acid of the penem product. The formation of alkali metal salts and metabolisable esters can be carried out by treating the free acid according to conventional procedures. For example, the free acid can be reacted with a stoichiometric amount of a suitable non-toxic base in an inert solvent followed by recovery of the desired salt by lyophilization or precipitation. Or a metabolisable ester can be formed by reaction of an alkali metal salt of a penem with a reactive derivate of the desired ester function; for example, the phthalidyl or pivaloyloxymethyl esters are preparable by reaction of the corresponding alkali metal penem salts with chlorophthalide or pivaloyloxymethylchloride in a solvent such as dimethylformamide, preferably with the addition of a catalytic amount of sodium iodide.

### REACTION SCHEME

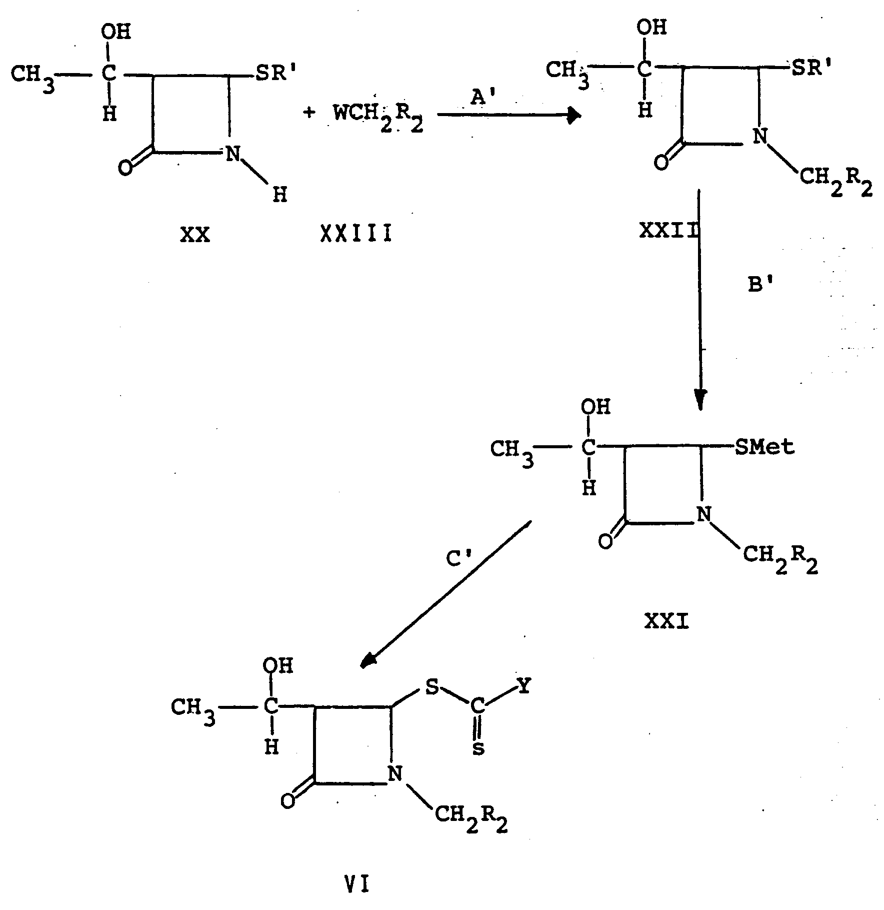

The intermediates of formula VI can be made, for example, by following the reaction scheme as shown in the accompanying flow chart. As in the case of reaction scheme 1 described above, it will be understood that any functional groups in any of the intermediates will be protected if necessary, or desired.

### Step A'

The reaction of azetidinone XX with the α-substituted acetate XXIII is preferably carried out in the presence of an acid acceptor, preferably at a temperature in the range from about 15°C to about 30°C.

W is a leaving group preferably tosyl, mesyl, chloro, bromo, iodo or trifluoromethylsulfonyl, most preferably iodo or bromo.

R₂ in the acetate XXIII is preferably allylic-oxycarbonyl most preferably allyloxycarbonyl.

If the solvent is also an acid acceptor, e.g. pyridine, no additional acid acceptor is required. Alternatively, an organic solvent which is not an acid acceptor, e.g. acetonitrile may be employed. In these cases, a separate acid acceptor, organic or inorganic must be used. Preferably the reaction is conducted in acetonitrile using cesium carbonate or tetraalkylammonium hydroxide as the acid acceptor.

Step B' can be carried out by following the procedures described above in connection with step D of reaction scheme 1.

Step C' can be carried out in a suitable solvent. The temperature will usually be in the range of about 10°C to about 45°C; about 25°C is preferred.

Usually the metal salt intermediate of formula XXI will be treated to protect the 5 position hydroxy group (if it is not already protected) before step C' is carried out. The resulting protected group can be deprotected, if desired, after the cyclization of the compound of formula VI i.e. the deprotection being carried out for example, on the compound of formula V. Conventional hydroxy protection and deprotection procedures can be used as discussed below.

In the preferred form of this process the intermediate of formula XXII is utilized directly in the reaction of step B', without separation from the reaction mixture in which it is formed (in step A') and the compound of formula XXI so formed likewise treated, directly, without isolation from the reaction mixture in the procedure of step C'.

If the intermediate of formula XXII formed in step B is treated to protect the 5 position hydroxy group before it is treated in step C' the protecting procedure can likewise be carried out directly on the intermediate of formula XXI without separating it from the reaction mixture in which it is formed. And likewise the protected intermediate so formed can be treated in step C' without separation from the reaction mixture.

Similarly the cyclization of intermediate of formula VI and the removal of any 8 position hydroxy protecting group from the resulting compound can be carried out sequentially on intermediates without separating the cyclized product from the reaction mixtures in which they are formed, where the 8-hydroxy group in the resulting penem is protected, this will then be removed by conventional procedures. Subsequent deprotection of the 3 position carboxyl group and formation of a free acid, salt or metabolisable ester can be carried out using the procedures discussed above.

Suitable hydroxy protecting groups are well known in the penem art and methods for their attachement to hydroxy groups are likewise well known. A particularly preferred protecting procedure for instance, for the protection of the 5-hydroxy group in the compounds of formula XXI comprises reacting the appropriate intermediate, (e.g. of formula XXI) having an unprotected hydroxy group, with bis-silylacetamide which readily forms the trimethylsilyl protecting group at the appropriate hydroxy moiety. The protection of the 5-hydroxy moiety in the azetidinone intermediates in the processes discussed above can be carried out without isolation of the intermediate involved. Thus the inert solvent used may be the same as the one used in the preceding step e.g. DMF. Other solvents for example, chloroform and methylene chloride may also be used. Temperatures for the hydroxy protection procedure are typically from about 0°C to about 30°C.

Methods for the removal of a group protecting 5 or 8-hydroxy group are well known in the penem art. Preferably, when the hydroxy protecting group is trimethylsilyl, addition of a mild aqueous acid solution, such as acetic acid, to the solution in which the intermediate to be deprotected is prepared effects removal. Thus, for example, there is no need to isolate a protected derivative of the compound of formula IV before proceeding to deprotect the 8-hydroxy group.

The following preparations, examples and illustrations describe in detail the processes of the present invention, methods for the preparation of the starting material and illustrations of the use of the intermediates produced by the instant process. Throughout these preparations, examples and illustrations, "NMR" denotes nuclear magnetic resonance spectra; "rotation" denotes optical rotation of the compounds in a suitable solvent; "MS" denotes mass spectra; UV denotes ultraviolet spectra; "IR" denotes infrared spectra: Chromatography is performed on silica gel unless otherwise noted. The term "room temperature" refers to about 18°C to about 25°C.

### PREPARATION OF STARTING MATERIALS

### PREPARATION A

### (3S,4R,5R)-3-(1-Trichlorethoxycarbonyloxyethyl)-4-(tri phenylmethylthio)azetidin-2-one

To a 250ml flask add 7.8 grams (0.0223M) of 3-(1-trichloroethoxycarbonyloxyethyl)-4-acetoxyazetidin-2-one, 220ml acetonitrile, 2.6 grams (0.252M) cesium carbonate, and 5.2 grams (0.0188M) triphenylmethanethiol (tritylthiol). After stirring for 5 hours, add an additional 1.0 gram (0.0036M) triphenylmethanethiol and stir the mixture for another one-half hour. After overnight refrigeration filter to remove the solids and remove the solvents under reduced pressure to yield a crude reaction product. Chromatograph this crude product on coarse silica gel eluting with methylene chloride changing to 10% and 20% ethyl acetate/methylene chloride to afford 7.89 grams (3S,4R,5R)-3-(1-trichloroethoxycarbonyloxyethyl)-4-(triphenylmethylthio) azetidin-2-one with spectra as follows:
- NMR: =: 7.7-7.1,16H; 5.05,1H,m; 4.85,2H,q (J=18Hz); 4.45,1H,d(J=1.5Hz); 3.3, 1H,dd(J=1.5,9Hz); 1.5,3H,d(J=9Hz).

### PREPARATION B

### 1-METHYL-2-CHLOROMETHYL-IMIDAZOLE

Charge 10 grams of 1-methyl imidazole and 100 ml of a 37% aqueous formaldehyde solution to a 150 ml Parr bomb and heat to 125°C. in an oil bath. Remove the water and evaporate the residue to a gel. Extract the gel in solution with methanol. Remove the methanol. Isolate from a coarse silica column, the product, 1-methyl-2-hydroxymethyl-imidazole, and crystallize from CCl₄. Mix 4.4 gram of 1-methyl-2-hydroxymethyl-imidazole with 5.7 ml SOCl₂ in 50 ml CHCl₃ in a reaction flask. Stir for 18 hours and remove the solvent and excess SOCl₂ under vacuum. Evaporate to dryness to recover the product, 1-methyl-2-chloromethyl-imidazole.

### PREPARATION C

### 2-(N-ALLYLOXYCARBONYLGLYCLAMINO)-ETHANETHIOL

Add pivaloyl chloride (2.4 ml) in CH₂Cl₂ (10 ml) to a cooled (0°-5°C.) and stirred solution of N-allyloxycarbonylglycine (3.18 g) and triethylamine (2.8 ml) in dry CH₂Cl₂ (50 ml). Stir the mixture at 0°-5°C. for 15 mins. and then add a solution of 2-aminoethanethiol hydrochloride (2.4 g) and triethylamine (2.8 ml) in ethanol (15 ml) and CH₂Cl₂ (40 ml). Stir at room temperature for 1 hour, wash the mixture with aqueous 2N-H₂SO₄ and aqueous NaHCO₃, dry and evaporate. Triturate the resulting solid with ether, filter and dry to give the title compound.
PMR (CDCl₃): δ 1.42 (t, J=8 Hz, exch. by D₂O 1H), 2.61 (m, 2H), 3.50 (q, J=7 Hz, 2H), 3.88 (d, J=7Hz, 2H), 4.57 (m, 2H), 5.1-5.5 (m, 2H), 5.6-6.2 (m, 2H; 1H exch. by D₂O) and 7.0 (br. S, 1H, exch.by D₂O).

### EXAMPLE 1

### Preparation of Allyl-(5R,6S,8R)-2-thiol-6-(1-hydroxy-ethyl) penem-3-carboxylate and Allyl (5R,6S,8R)-2-thione 6-(1-hydroxyethyl)penam-3-carboxylate

### A) Preparation of (3S,4R,5R)-1-allyloxycarbonylmethyl)-3-(1-hydroxyethyl)-4-(triphenylmethylthio)azetidin-2-one

Add 3gm of (3S,4R,5R)-3-(1-hydroxyethyl)-4-(triphenylmethylthio)azetidin-2-one to 10ml of acetonitrile containing 0.286gm of cesium carbonate. Add 0.2gm of α-iodo allyl acetate to the system. Stir the system at room temperature for 16 hours. Dilute with ether (50ml), filter and wash the ether layer with 1% aqueous phosphoric acid, followed by water. After drying over sodium sulfate remove solvent to give a foamy solid.
- NMR: =: 8.4, 1H, s; 7.65, 1H, d(J=1Hz); 7.05, 1H d(J=1Hz); 5.95, 1H, d (J=2Hz); 5.8, 1H, m; 5.45-5.1, 2H, m; 4.3, 1H, m; 4.1, 2H, Q(J=16Hz); 3.5, d d(J=2,6); 1.35; 3H, d(J=6Hz).

### B) Preparation of Silver (3S,4R,5R)-3-(1-hydroxyethyl)-1-allyloxycarbonylmethyl-azetidin-2-one-4-thiolate

To a 50ml flask having a nitrogen atmosphere add 10ml of methanol and 460mg of (3S,4R,5R)-1-(allyloxycarbonyl)-3-(1-hydroxyethyl)-4-(triphenylmethylthio)azetidin-2-one. To this system add 160mg silver nitrate and 0.15ml of pyridine. Stir the system at 20°C for 1 hour. Stop the reaction and remove the methanol by stripping to give the title compound.
B') Repeat the procedure of step B but replacing the silver nitrate with an equivalent quantity of -
   1) thallic nitrate,
   2) cupric nitrate and
   3) Hg(NO₃)₂.H₂O
   to obtain respectively thallium (3S,4R,5R)-3-(1-hydroxyethyl)-1-allyloxycarbonylmethyl-azetidin-2-one-4-thiolate,
   copper (3S,4R,5R)-3-(1-hydroxyethyl)-1-allyloxycarbonylmethyl-azetidin-2-one-4-thiolate, and
   mercury (3S,4R,5R)-3-(1-hydroxyethyl)-1-allyloxycarbonylmethyl-azetidin-2-one-4-thiolate.

### C) Preparation of Silver (3S,4R,5R)-3-(1-trimethylsilyloxyethyl)-1-allyloxycarbonylmethyl-azetidin-2-one-4-thiolate

Add the entire amount of silver (3S,4R,5R)-3-(1-hydroxyethyl)-1-allyloxycarbonyl-methylazetidin-2-one-4-thiolate produced in step (B) above to 25ml of methylene chloride. To this system add 1.1ml of bis-silylacetamide. Stir the system at room temperature for 15 minutes to give the title compound.
C') Repeat the procedure of step C but replacing the silver bait with the respective thallium, copper and mercury salts obtained in step B' to afford respectively
   thallium (3S,4R,5R)-3-(1-trimethylsilyloxyethyl)-1-allyloxycarbonylmethyl-azetidin-2-one-4-thiolate,
   copper (3S,4R,5R)-3-(1-trimethylsilyloxyethyl)-1-allyloxycarbonylmethyl-azetidin-2-one-4-thiolate, and
   mercury (3S,4R,5R)-3-6)-trimethylsilyloxyethyl)-1-allyloxycarbonylmethyl-azetidin-2-one-4-thiolate.

### D) Preparation of (3S,4R,5R)-1-(allyloxycarbonylmethyl)-3-(1-trimethylsilyloxyethyl)-4-(1'-imidazolylthiocarbonylthio)azetidin-2-one

After completion of step (C) above and to the same solution add 350mg of thiocarbonyldiimidazole. Stir the system at room temperature for 3 hours. Filter the solution and wash the precipitate with methylene chloride. Collect the filtrate and remove the methylene chloride by stripping. Chromatograph the residue on silica gel eluting with 20% ethyl acetate/methylene chloride to yield 335mg of the title compound.
D') Repeat the procedure of step D but using the respective thallium, copper and mercury salts produced in step C' to afford in each case the title compound.

### E) Preparation of (5R,6S,8R) allyl-2-thiol-6-(1-trimethylsilyloxymethyl)penem-3-carboxylate and (5R,6S,8R) allyl-2-thione-6-(1-trimethylsilyloxyethyl)penam equilibrium mixture

Add 170mg of (3S,4R,5R)-1-(allyloxycarbonylmethyl) -3-(1-trimethylsilyloxymethyl)-4-(1'-imidazolyl-thiocarbonylthio)azetidin-2-one to 40ml of anhydrous tetrahydrofuran under a nitrogen atmosphere. Cool the system to -78°C. and then add 0.6ml of 1 M lithium di-(trimethylsilyl) amine in hexane dropwise to the system. Stir the system at -78°C. for 5 minutes. Add 0.2ml of acetic acid to the system. Dilute the system to 200ml with methylene chloride. Wash the organic solution with water, aqueous sodium bicarbonate solution and again with water. Purify the product by chromatography by rapidly eluting the sample through silica gel with 5% ethyl acetate/methylene chloride to afford 125mg of the desired products and the desilylated products.

### F) Preparation of (5R,6S,8R) Allyl-2-thiol-6-(1-hydroxyethyl)penem-3-carboxylate and (5R,6S,8R) Allyl-2-thione 6-(1-hydroxyethyl)penam equilibrium mixture

To a 25ml flask add the entire mixture produced in step (E) along with 5ml of tetrahydrofuran, 1ml of water and 1ml of acetic acid. Stir the system at room temperature for 2 hours. Add ethyl acetate to the solution and wash the organic phase with sodium bicarbonate solution, water and then brine. Dry the organic phase over anhydrous sodium sulfate, filter and remove the solvent by stripping to give the title compound.

Thereafter the product of step F can be treated, if desired by the procedure described in step I of the Example 7 to yield allyl (5R,6S,8R)-2-ethylthio-6-(1-hydroxyethyl)penem 3-carboxylate.

### EXAMPLE 2

### Preparation of (5R,6S,8R) Allyl-2-(2',2',2'-trifluoroethyl) -6-(1-hydroxyethyl)penem-3-carboxylate

1. Dissolve 0.735ml pyridine in 25ml dry toluene and cool to -20°C. under nitrogen. Add 1.45ml trifluoromethanesulfonic anhydride followed by 0.703ml 2,2,2-trifluoroethanol and allow to warm to room temperature. Wash the resultant residue with water, dry with anhydrous sodium sulfate and distill, collecting all fractions with a boiling point less than 100°C to obtain trifluoromethyl 2,2,2-trifluoroethylsulfonate.
2. Add (5R,6S,8R) allyl-2-thiol-6-(1-hydroxyethyl)penem-3-carboxylate, (5R,6S,8R) allyl-2-thione-6-(1-hydroxyethyl)penam-3-carboxylate equilibrium mixture (produced by following the procedure of Example 7 up to step H) to 3ml of tetrahydrofuran and 5ml of trifluoromethyl 2,2,2-trifluoroethylsulfonate Add 1 equivalent of potassium carbonate (powder) to the system. Maintain the reaction mixture at room temperature for 1-1/2 hours and then store the solution in a refrigerator overnight. Remove the solution from the refrigerator and stir at room temperature for 1 hour. Filter the solution and wash with methylene chloride/2% phosphoric acid. Remove the solvent by evaporation. Dissolve the residue in warm 1:1 chloroform:petroleum ether and cool. The product crystallizes from solution to yield 168 mg of the title compound.

### EXAMPLE 3

### Sodium-(5R,6S,8R)-2-(2,3-dihydroxy-1-propylthio)-6-(1-hydroxyethyl)-penem-3-carboxylate

A. 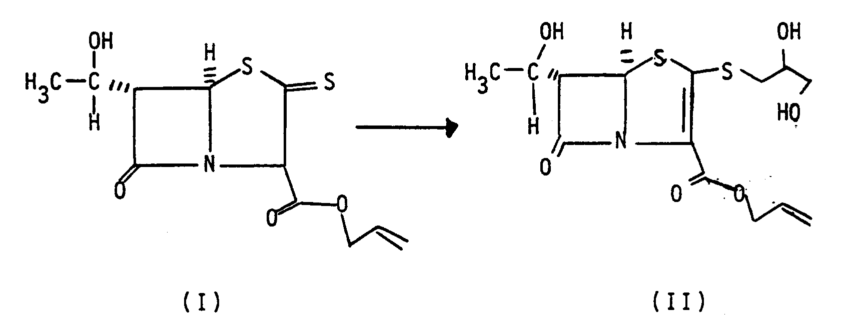 Add to 100 ml flask 400 mg (0.000696 M) of an equilibrium mixture of thione I with its corresponding thiol tautomer (Example 7, step H), 10 ml distilled tetrahydrofuran, 154 mg (0.00208 M) (3 eq.) of glycidol and 25 mg potassium carbonate. Stir until thin layer chromatography indicates the absence of starting material. Acidify with 10% phosphoric acid up to pH7, remove the tetrahydrofuran on a rotary evaporator and chromatograph the residue on a coarse silica gel column with 100% ethyl acetate to yield, after isolation, 160 mg of compound II (61% yield).
B. To a 50 ml flask add, under a nitrogen atmosphere a solution of the 160 mg of compound II obtained in step A in 2 ml ethyl acetate, 73 mg (0.000442 M) sodium-ethyl-2-hexanoate, 10 mg triphenylphosphine and 5 mg Pd(Ph₃)P₄. Stir the reaction mixture for 3 hours, re-frigerate overnight, add water and extract the product on a HPLC column with 100% H₂O, to yield, after isolation 32 mg of the title compound as a white amorphous solid.

### EXAMPLE 4

### Sodium-(5R,6S,8R)-2-(2,3-dihydroxypropylthio)-6-(1-hydroxyethyl)-penem-3-carboxylate

A. To a solution of 0.20 g of an equilibrium mixture of allyl-(5R,6S,8R)-6-(1-hydroxyethyl)-2-thione and allyl-(5R,6S,8R)-6-(1-hydroxyethyl)-2-thiol-penem-3-carboxylate and diisopropylethylamine (0.15 g) in dry acetonitrile (4 ml) add 2,3-dihydroxy-1-bromopropane (0.2 g). After about 1 hour at 25°C. dilute with aqueous tartaric acid and dry over magnesium sulfate. Evaporate and isolate the residue from dichloromethane to give allyl (5R,6S,8R)-6-(1-hydroxyethyl)-2-(2,3-dihydroxypropylthio)-penem-3-carboxylate.
B. Treat 160 mg of the product compound from step A to exactly the same procedure as in Example 3B to yield after isolation 32 mg of the title compound.

### EXAMPLE 5

### (5R,6S,8R)-2-(1-Methyl-2-imidazolylmethylthio)-6-(1-hydroxyethyl)-penem-3-carboxylic acid

### A. (5R,6S,8R)-Allyl-2-(1-methyl-2-imidazolylmethylthio)-6-(1-hydroxyethyl)-penem-3-carboxylate

Charge to a 250 ml flask under nitrogen 1.38 gram of the thione-thiol equilibrium mixture of Example 7, step H, 50 ml of tetrahydrofuran (THF) and 1.2 gram of 1-methyl-2-chloromethyl-imidazole (preparation B). Cool to 0°C. Add dropwise over 3 minutes 1.15 gram of NaHCO₃ as a 10% aqueous solution. Stir for about 45 minutes and let stand for one hour at 0°C. Remove the THF solvent and recover the title product on a silica column.
- NMR - (CDCl₃) δ =: 6.95, 1H,s; 6.86, 1H, s; 5.9, 1H, m; 5.73, 1H, d; 5.32, 2H, m; 4.7, 2H, m; 4.3, 2H, s; 4.2, 1H, m; 3.7, 1H, dd (J=1.5, 6Hz); 3.68, 3H, s; 1.3, 3H,d (J=6 Hz).

B. Dissolve 1.05 grams of the product of step A in 40 ml of the ethyl acetate. React at room temperature with about 200 mg Pd° reagent, 200 mg triphenyl phosphine and 1.5 ml hexanoic acid. Extract the resulting title compound with water. Increase the yield by dissolving unreacted starting material in 15 ml CH₂Cl₂, 1 ml hexanoic acid, 0.3 ml pyridine, 1.50 mg triphenyl phosphine and about 100 mg Pd° for 1/2 hour. Extract the resulting title compound with water and combine with the previous extract - the product has
   - NMR - (D₂O) =: 7.3, 1H, s; 7.27, 1H, s; 5.6, 1H, d(J=1.6 Hz); 4.4, 1H, m; 3.85, 1H, dd(J=1.5, 6Hz); 3.81, 3H, s; 1.24, 3H, d(J=6 Hz).

### EXAMPLE 6

A. 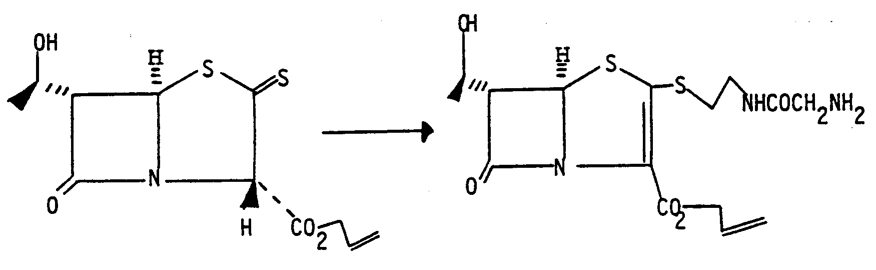 To a solution of 0.20 g of an equilibrium mixture of allyl (5R,6S,8R)-6-(1-hydroxyethyl)-2-thione-penam-3-carboxylate and allyl (5R,6S,8R)-6-(1-hydroxyethyl)-2-thiol-penem-3-carboxylate and diisopropylethylamine (0.15 g) in dry acetonitrile (4 ml) add 1-iodo, 2-(N-allyloxycarbonylglycylamino)-ethane (0.23 g). After 1 hour at room temperature dilute with aqueous tartaric acid and dry over magnesium sulfate. Evaporate and isolate the residue from dichloromethane to give allyl (5R,6S,8R)-6-(1-hydroxyethyl)-2-( 2[N-allyloxycarbonylglycylamino]ethylthio)-penem-3-carboxylate, as a pale yellow foam.
B. Treat the product from step A by following exactly the procedure described in Example 17, step B, to obtain (5R,6S,8R)-6-(1-hydroxyethyl)-2-(glycylaminoethylthio)-penem-3-carboxylic acid.

### EXAMPLE 7

### Allyl-(5R,6S,8R)-6-(1-hydroxyethyl)-2-[2-methylcarbamoyl)ethylthio]-penem-3-carboxylate

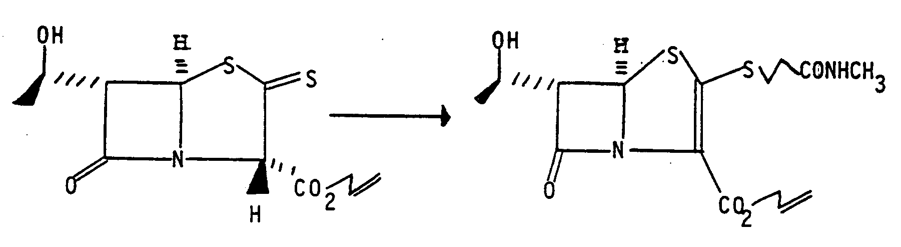

To a solution of 0.20 g of an equilibrium mixture of allyl (5R,6S,8R)-6-(1-hydroxyethyl)-2-thione-penem-3-carboxylate and allyl (5R,6S,8R)-6-(1-hydroxyethyl)-2-thiol-penem-3-carboxylate, and diisopropylethylamine (0.15 g) in dry acetonitrile (4 ml) add N-methyl-3-iodopropionamide (0.2 g). After 1 hour at 25°C., dilute with ethyl acetate (25 ml), wash with aqueous tartaric acid and dry (MgSO₄). Evaporate and crystallise the residue from dichloromethane to give the title compound.

## Claims

1. A process for the preparation of compounds of the formula I 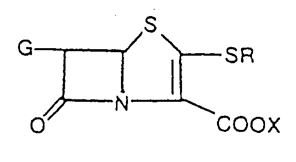 having the stereo configuration 5R,6S, wherein R is hydrogen; lower alkyl; loweralkyl substituted with one or more substituents independently selected from halogen, hydroxy, lower alkoxy, cyano, oxo, carb(lower)alkoxy, carbamoyl, N-lower-alkyl-substituted-carbamoyl, N,N-diloweralkyl substituted-carbamoyl, amino, loweralkylamino, diloweralkylamino, lower alkanoylamino, aminoacylamino, alkylthio, arylthio, heterocyclic, heterocyclicthio, 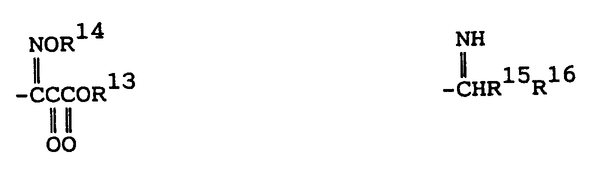 (wherein R¹⁴ and R¹³ are independently hydrogen or lower alkyl and R¹⁵ and R¹⁶ are each lower alkyl or R¹⁵ and R¹⁶ together with the nitrogen to which they are attached form a heterocyclic ring of 3 to 6 members) butanolidyl, aryl, heteroaryl or aryl or heteroaryl substituted with 1 to 4 substituents independently selected from loweralkyl, hydroxy, lower-alkoxy, halogen, haloloweralkyl, loweralkylthio, amino, lower-alkylamino, diloweralkylamino, carboxyloweralkyl, nitro, and cyano;
aryl or aryl substituted with 1 to 4 substituents independently selected from loweralkyl, hydroxy, lower alkoxy, halogen, haloloweralkyl, loweralkylthio, amino, loweralkylamino, diloweralkylamino, carboxyloweralkyl, nitro, cyano, aminoalkyl and carboxy; and
heteroaryl or heteroaryl substituted with 1 to 4 substituents independently selected from loweralkyl, hydroxy, lower alkoxy, halogen, haloloweralkyl, lower alkylthio, loweralkylsulfonyl, amino, loweralkylamino, diloweralkylamino, carboxyloweralkyl, nitro and cyano,
wherein the term "halogen" means fluoro, chloro and bromo,
the term "loweralkanoylamino" means the group 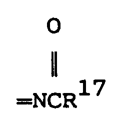 wherein R¹⁷ is lower alkyl,
the term "aryl" means aromatic groups of 6 to 10 carbons,
the term "heterocyclic" means cyclic groups of 5 or 6 ring atoms wherein 1 to 4 ring atoms are selected from nitrogen, sulfur and oxygen,
the term "heteroaryl" means aromatic heterocyclic groups of 5 to 10 ring atoms wherein 1 to 3 ring atoms are selected from the group consisting of nitrogen, sulfur and oxygen,
and the term loweralkyl means branched and straight-chain alkyl groups of 1 to 6 carbon atoms,
G is hydroxy C₁ to C₆ alkyl, X is hydrogen, a pharmaceutically acceptable salt forming group, a pharmaceutically acceptable ester group or a carboxy protecting group, and the tautomers thereof when R is hydrogen, characterised in that a compound of formula VI 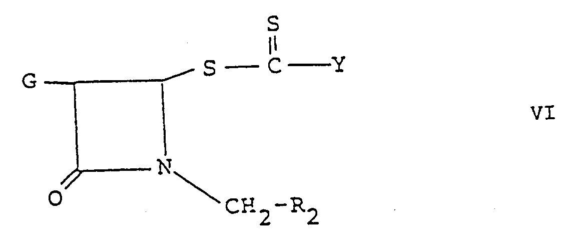 having appropriate stereo configuration, wherein R₂ is a protected carboxy group and Y is a leaving group, is subjected to intramolecular cyclisation in an anhydrous inert organic solvent in the presence of non-nucleophilic strong base whereby a tautomeric compound of formulas Va and Vb is obtained 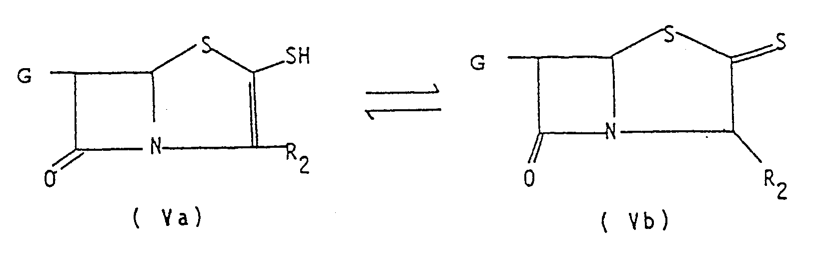 having the stereo configuration 5R, 6S, in which R₂ is as defined above, and that, if desired, such tautomeric compound is transformed into a compound of formula I wherein R is other than hydrogen by introducing such R by known conventional methods.

2. A process according to Claim 1 wherein R is hydrogen, lower alkyl, -CH₂-CF₃, -CH₂-CH₂F, -CH₂-CH(OH)-CH₂-OH, -CH₂-CH₂-OH, -1-methylimidazolyl-2-yl-methyl, -CH₂-CH₂-NH-CO-CH₂-NH₂ -CH₂-CH₂-CO-NH-CH₃, -CH₂-CH₂-NH₂, benzyl, -CH₂CH₂-Cl, phenylthioethyl, 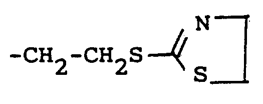 cyanomethyl, -CH₂-CO-CH₃, -CH₂CO₂CH₃, -CH₂-CH₂-CN, 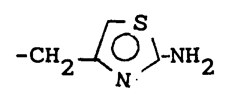 -CH₂COONa, -CH₂CH₂COONa, -CH₂-CH(OH)COONa, -CH₂-CH (OH)-CH₃, -CH₂-C(NH)-N(CH₃)₂,

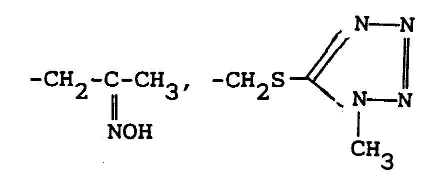

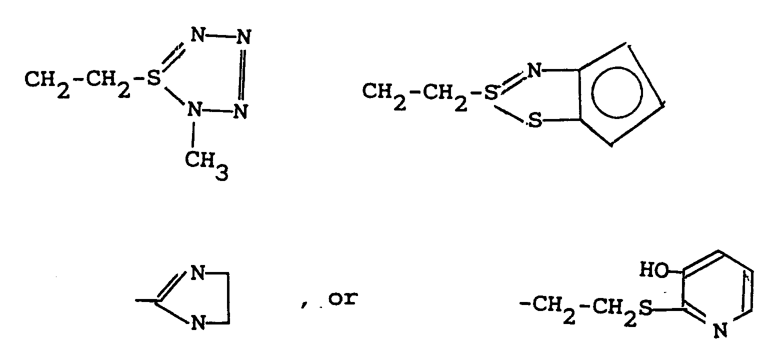

3. A process according to Claim 1 or Claim 2 characterized in that R₂ is allyloxycarbonyl.

4. A process according to any preceding claim, characterized in that a compound of formula I in which G is 1-hydroxyethyl and having the stereochemistry (5R,6S,8R) is produced.

5. A compound of the formula 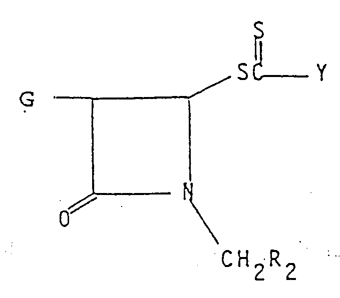 in which G is hydroxy C₁ to C₆ alkyl, Y is a leaving group, R₂ is a protected carboxy group, and the hydroxy group in G may be protected.

6. A tautomeric compound of the formulas 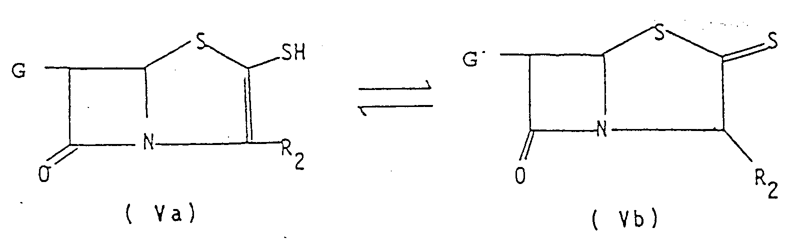 in which G is as defined in Claim 1 and R₂ is allyloxy carboxyl.

## Patentansprüche

1. Verfahren zur Herstellung von Verbindungen der Formel I 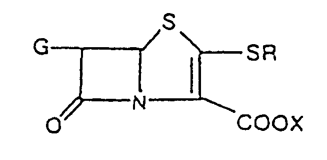 mit der Stereokonfiguration 5R,6S, worin R folgendes ist: Wasserstoff, Niederalkyl, Niederalkyl, das mit einem oder mehreren Substituenten substituiert ist, die unabhängig ausgewählt sind aus Halogen, Hydroxy, Niederalkoxy, Cyano, Oxo, Carb(nieder)alkoxy, Carbamoyl, N-Niederalkyl-substituiertes-Carbamoyl, N,N-Di(niederalkyl)-substituiertes-Carbamoyl, Amino, Niederalkylamino, Di(niederalkyl)amino, Niederalkanoylamino, Aminoacylamino, Alkylthio, Arylthio, heterocyclisch, heterocyclisch-Thio, 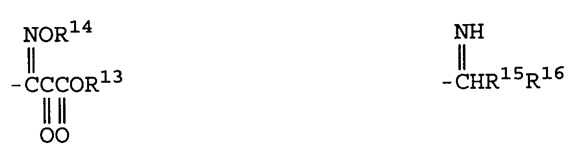 (worin R¹⁴ und R¹³ unabhängig Wasserstoff oder Niederalkyl sind und R¹⁵ und R¹⁶ beide Niederalkyl sind oder R¹⁵ und R¹⁶ zusammen mit dem Stickstoff, an den sie gebunden sind, einen heterocyclischen, 3- bis 6-gliedrigen Ring bilden), Butanolidyl, Aryl, Heteroaryl oder Aryl oder Heteroaryl, das mit 1 bis 4 Substituenten substituiert ist, die unabhängig ausgewählt sind von Niederalkyl, Hydroxy, Niederalkoxy, Halogen, Halogenniederalkyl, Niederalkylthio, Amino, Niederalkylamino, Di(niederalkyl)amino, Carboxyniederalkyl, Nitro und Cyano; Aryl oder Aryl, das mit 1 bis 4 Substituenten substituiert ist, die unabhängig ausgewählt sind von Niederalkyl, Hydroxy, Niederalkoxy, Halogen, Halogenniederalkyl, Niederalkylthio, Amino, Niederalkylamino, Di(niederalkyl)amino, Carboxyniederalkyl, Nitro, Cyano, Aminoalkyl und Carboxy; sowie Heteroaryl oder Heteroaryl, das mit 1 bis 4 Substituenten substituiert ist, die unabhängig ausgewählt sind von Niederalkyl, Hydroxy, Niederalkoxy, Halogen, Halogenniederalkyl, Niederalkylthio, Niederalkylsulfonyl, Amino, Niederalkylamino, Di(niederalkyl)amino, Carboxyniederalkyl, Nitro und Cyano,
wobei der Ausdruck "Halogen" Fluor, Chlor und Brom bedeutet,
der Ausdruck "Niederalkanoylamino" die Gruppe 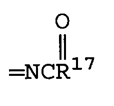 bedeutet, wobei R¹⁷ Niederalkyl ist,
der Ausdruck "Aryl" aromatische Gruppen mit 6 bis 10 Kohlenstoffatomen bedeutet,
der Ausdruck "heterocyclisch" cyclische Gruppen mit 5 oder 6 Ringatomen bedeutet, wobei 1 bis 4 Ringatome aus Stickstoff, Schwefel und Sauerstoff ausgewählt sind,
der Ausdruck "Heteroaryl" aromatische heterocyclische Gruppen mit 5 bis 10 Ringatomen bedeutet, wobei 1 bis 3 Ringatome aus der aus Stickstoff, Schwefel und Sauerstoff bestehenden Gruppe ausgewählt sind,
und der Ausdruck Niederalkyl verzweigte und geradkettige Alkylgruppen mit 1 bis 6 Kohlenstoffatomen bedeutet,
G Hydroxy-C₁- bis -C₆-Alkyl ist, X Wasserstoff, eine Gruppe, die pharmazeutisch akzeptable Salze bildet, eine pharmazeutisch akzeptable Estergruppe oder eine Schutzgruppe für Carboxyfunktionen ist, sowie die Tautomeren davon, wenn R Wasserstoff ist, dadurch gekennzeichnet, daß eine Verbindung der Formel VI 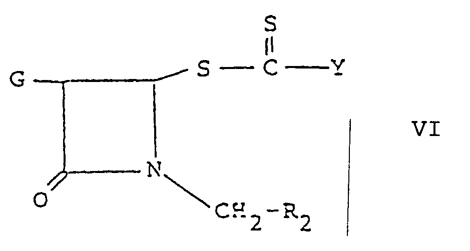 mit einer geeigneten Stereokonfiguration, worin R₂ eine geschützte Carboxygruppe und Y eine Abgangsgruppe ist, intramolekular cyclisiert wird in einem nichtwäßrigen inerten organischen Lösungsmittel in Gegenwart von nichtnucleophiler starker Base, wodurch eine tautomere Verbindung der Formeln Va und Vb erhalten wird 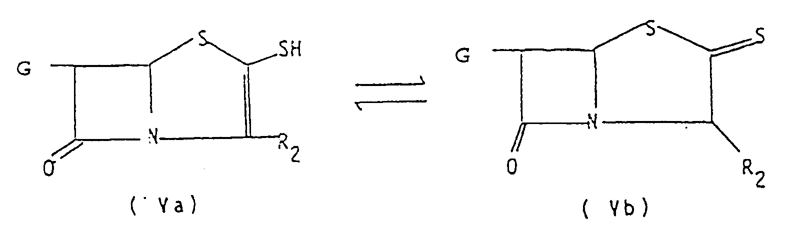 mit der Stereokonfiguration 5R,6S, worin R₂ wie oben definiert ist, und daß, falls gewünscht, diese tautomere Verbindung zu einer Verbindung der Formel I umgesetzt wird, worin R nicht Wasserstoff ist, indem man ein solches R durch bekannte konventionelle Methoden einführt.

2. Verfahren gemäß Anspruch 1, wobei R folgendes ist: Wasserstoff, Niederalkyl, -CH₂-CF₃, -CH₂-CH₂F, -CH₂-CH(OH)-CH₂-OH, -CH₂-CH₂-OH, -1-Methylimidazolyl-2-yl-methyl, -CH₂-CH₂-NH-CO-CH₂-NH₂, -CH₂-CH₂-CO-NH-CH₃, -CH₂-CH₂-NH₂, Benzyl, -CH₂CH₂-Cl, Phenylthioethyl, 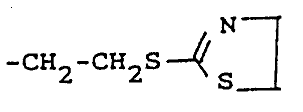 Cyanomethyl, -CH₂-CO-CH₃, -CH₂CO₂CH₃, -CH₂-CH₂-CN, 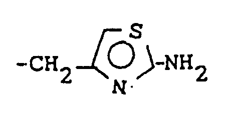 -CH₂COONa, -CH₂CH₂COONa, -CH₂-CH(OH)COONa, -CH₂-CH(OH)-CH₃, -CH₂-C(NH)-N(CH₃)₂,

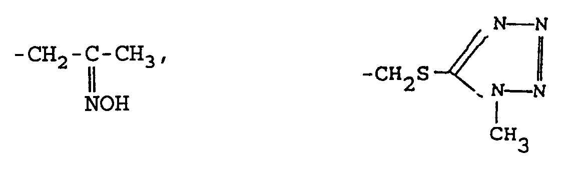

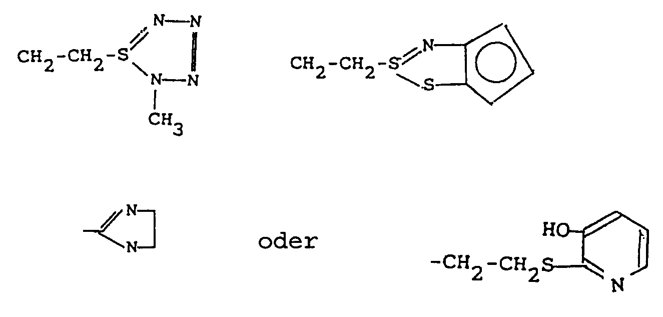

3. Verfahren gemäß Anspruch 1 oder Anspruch 2, dadurch gekennzeichnet, daß R₂ Allyloxycarbonyl ist.

4. Verfahren gemäß einem der vorhergehenden Ansprüche, dadurch gekennzeichnet, daß eine Verbindung der Formel I, in der G 1-Hydroxyethyl ist und die die Stereochemie (5R,6S,8R) besitzt, erzeugt wird.

5. Eine Verbindung der Formel 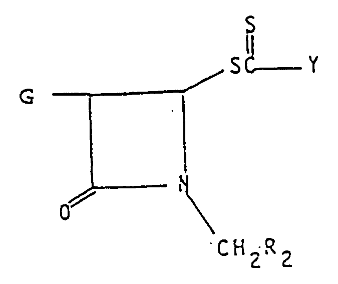 in der G Hydroxy-C₁- bis -C₆-Alkyl ist, Y eine Abgangsgruppe ist, R₂ eine geschützte Carboxygruppe ist und die Hydroxygruppe in G geschützt sein kann.

6. Eine tautomere Verbindung der Formeln 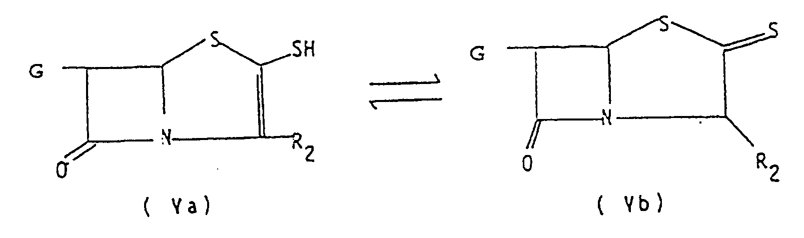 worin G wie in Anspruch 1 definiert ist und R₂ Allyloxycarboxyl ist.

## Revendications

1. Procédé de préparation de composés de la formule I 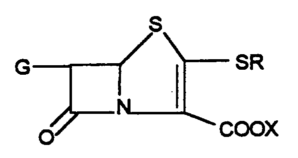 ayant la configuration stéréo 5R, 6S, où R est hydrogène; alkyle inférieur; alkyle inférieur substitué par un ou plusieurs substituants indépendamment choisis parmi halogène, hydroxy, alcoxy inférieur, cyano, oxo, carbalcoxy inférieur, carbamoyle, carbamoyle N-alkyle-inférieur-substitué, carbamoyle N,N-dialkyle inférieur-substitué, amino, alkylamino inférieur, dialkylamino inférieur, alcanoylamino inférieur, aminoacylamino, alkylthio, arylthio, hétérocyclique, thiohétérocyclique, 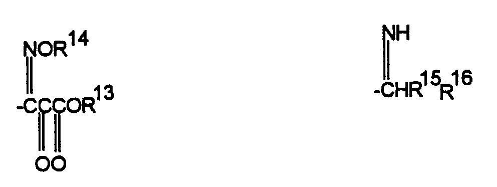 (où R¹⁴ et R¹³ sont indépendamment hydrogène ou alkyle inférieur et chacun de R¹⁵ et R¹⁶ est alkyle inférieur ou bien R¹⁵ et R¹⁶, avec l'azote auquel ils sont attachés, forment un noyau hétérocylique de 3 à 6 membres), butanolidyle, aryle, hétéroaryle ou aryle ou hétéroaryle substitué par 1 à 4 substituants indépendamment choisis parmi alkyle inférieur, hydroxy, alcoxy inférieur, halogène, haloalkyle inférieur, alkylthio inférieur, amino, alkylamino inférieur, dialkylamino inférieur, carboxyalkyle inférieur, nitro et cyano;
Aryle ou aryle substitué par 1 à 4 substituants indépendamment choisis parmi alkyle inférieur, hydroxy, alcoxy inférieur, halogène, haloalkyle inférieur, alkylthio inférieur, amino, alkylamino inférieur, dialkylamino inférieur, carboxyalkyle inférieur, nitro, cyano, aminoalkyle et carboxy; et
hétéroaryle ou hétéroaryle subsituté par 1 à 4 substituants indépendamment choisis parmi alkyle inférieur, hydroxy, alcoxy inférieur, halogène, haloalkyle inférieur, alkylthio inférieur, alkyle inférieur sulfonyle, amino, alkylamino inférieur, dialkylamino inférieur, carboxyalkyle inférieur, nitro et cyano,
où le terme "halogène" signifie fluoro, chloro et bromo,
le terme "alkanoylamino inférieur" signifie le groupe 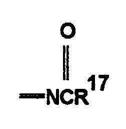 où R¹⁷est alkyle inférieur,
le terme "aryle" signifie des groupes aromatiques de 6 à 10 carbones,
le terme "hétérocyclique" signifie des groupes cycliques de 5 ou 6 atomes dans le noyau où 1 à 4 atomes du noyau sont choisis parmi azote, soufre et oxygène,
le terme "hétéroaryle" signifie des groupes hétérocycliques aromatiques de 5 à 10 atomes dans le noyau où 1 à 3 atomes dans le noyau sont sélectionnés dans le groupe consistant en azote, soufre et oxygène,
et le terme alkyle inférieur signifie des groupes alkyle à chaîne droite et ramifiée de 1 à 6 atomes de carbone,
G est hydroxy alkyle C₁ à C₆, X est hydrogène, un groupe formant un sel acceptable en pharmacie, un groupe ester acceptable en pharmacie, ou un groupe carboxy protecteur et leurs tautomères quand R est hydrogène, caractérisé en ce qu'un composé de formule VI 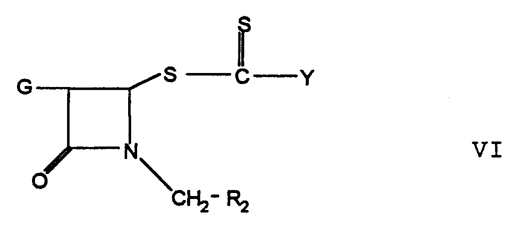 ayant la configuration stéréo appropriée, où R₂ est un groupe carboxy protégé et Y est un groupe partant, est soumis à une cyclisation intramoléculaire dans un solvant organique inerte anhydre en présence d'une base forte nonnucléophile pour ainsi obtenir un composé tautomère des formules Va et Vb 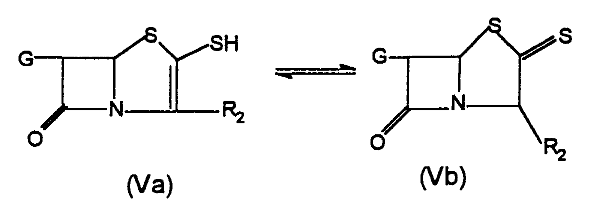 ayant la configuration stéréo 5R, 6S, où R₂ est tel que défini ci-dessus et en ce que, si on le souhaite, ledit composé tautomère est transformé en un composé de formule I où R est autre qu'hydrogène, par l'introduction dudit R par des méthodes conventionnelles connues.

2. Procédé selon la revendication 1, où R est hydrogène, alkyle inférieur, -CH₂ -CF₃, -CH₂-CH₂F, -CH₂-CH(OH)-CH₂-OH, -CH₂-CH₂-OH, -1-méthylimidazolyl-2-yl-méthyle, -CH₂-CH₂-NH-CO-CH₂-NH₂ -CH₂-CH₂-CO-NH-CH₃, -CH₂-CH₂-NH₂, benzyle, -CH₂CH₂-Cl, phénylthioéthyle, 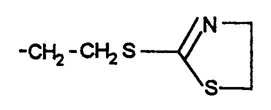 cyanométhyle, -CH₂ -CO-CH₃, -CH₂CO₂CH₃, -CH₂-CH₂-CN, 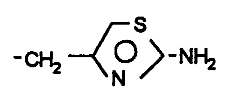 -CH₂COONa, -CH₂CH₂COONa, -CH₂-CH(OH)COONa, -CH₂ -CH (OH)-CH₃), -CH₂-C(NH)-N(CH₃)₂,

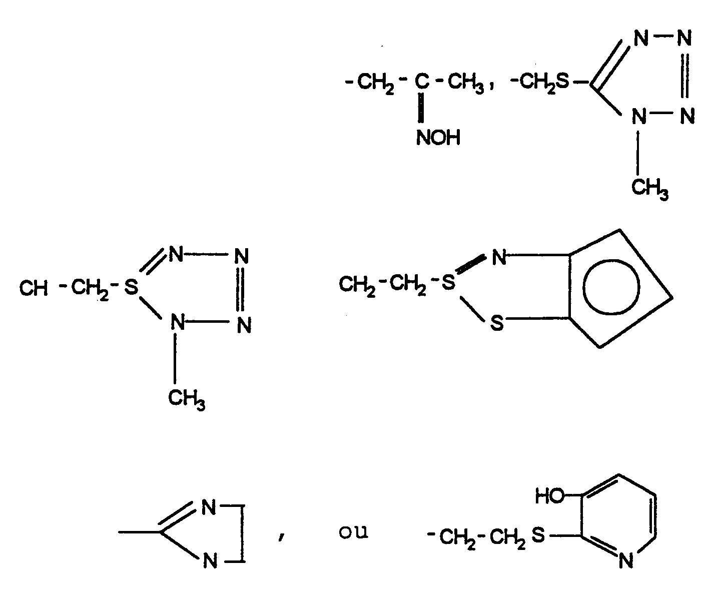

3. Procédé selon la revendication 1 ou la revendication 2, caractérisé en ce que R₂ est allyloxycarbonyle.

4. Procédé selon toute revendication précédente, caractérisé en ce qu'un composé de formule I où G est 1-hydroxyéthyle et qui a la stéréochimie (5R, 6S, 8R) est produit.

5. Composé de la formule 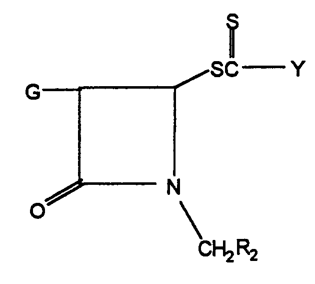 où G est hydroxy alkyle C₁ à C₆, Y est un groupe partant, R₂ est un groupe carboxy protégé et le groupe hydroxy dans G peut être protégé.

6. Composé tautomère des formules 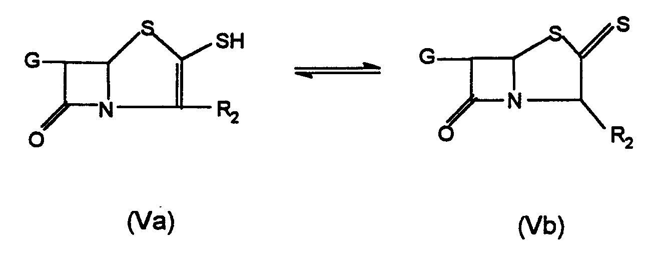 où G est tel que défini à la revendication 1 et R₂ est allyloxy carboxyle.
